(19) Europäisches Patentamt
European Patent Office
Office européen des brevets

(11) EP 0 713 694 B1

# (12) EUROPÄISCHE PATENTSCHRIFT

(45) Veröffentlichungstag und Bekanntmachung des Hinweises auf die Patenterteilung:
**01.12.1999 Patentblatt 1999/48**

(51) Int. Cl.[6]: **A61K 7/09**

(21) Anmeldenummer: **95110120.3**

(22) Anmeldetag: **29.06.1995**

## (54) Mittel zur dauerhaften Haarverformung

Permanent wave hair composition

Composition pour la déformation permanente des cheveux

(84) Benannte Vertragsstaaten:
**DE ES FR GB IT**

(30) Priorität: **24.11.1994 DE 4441873**

(43) Veröffentlichungstag der Anmeldung:
**29.05.1996 Patentblatt 1996/22**

(73) Patentinhaber:
**Wella Aktiengesellschaft**
**64274 Darmstadt (DE)**

(72) Erfinder:
- **Lang, Günther, Dr.**
  **D-64354 Reinheim (DE)**
- **Maresch, Gerhard**
  **D-64295 Darmstadt (DE)**

(56) Entgegenhaltungen:
**EP-A- 0 523 666**
**WO-A-90/03780**
**WO-A-91/10421**
**US-A- 4 139 610**
**US-A- 4 992 267**

EP 0 713 694 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft ein Mittel zur dauerhaften Haarverformung auf der Basis von Kondensationsprodukten aus Cystein und Glycin

Für eine schonende dauerhafte Verformung von geschädigtem, insbesondere gebleichtem oder gefärbtem Haar werden bevorzugt schwach sauer bis neutral eingestellte Verformungsmittel verwendet. Hierbei haben sich im Verlauf der letzten 30 Jahre die Thioglykolsäureester als für diesen Zweck am besten geeignete Reduktionsmittel erwiesen.

Dem Vorteil einer haarschonenden Verformungsbehandlung durch schwach saure bis neutrale Verformungsmittel stehen jedoch eine Reihe vor Nachteilen gegenüber. So besitzen diese Mittel eine im Vergleich zu mildalkalischen Verformungsmitteln auf Thioglykolatbasis geringere Wellwirksamkeit. Aus diesem Grunde ist für die Erzielung einer ausreichenden Umformung die Zufuhr von Wärme, eine Verlängerung der Einwirkungszeiten auf 20 bis 60 Minuten sowie die Verwendung von vergleichsweise dünnen Wicklern erforderlich. Eine Verwendung dieser Verformungsmittel für normales, nicht geschädigtes Naturhaar erscheint aufgrund der auch bei Wärmezufuhr erforderlichen langen Einwirkungszeiten von über 30 Minuten nicht sinnvoll, so daß die Anwendung von schwach sauer bis neutral eingestellten Verformungsmitteln bisher in der Regel auf vorgeschädigtes, leicht verformbares Haar beschränkt blieb

Ein weiterer erheblicher Nachteil von sauren Haarverformungsmitteln ist die schlechte Augen- und Hautverträglichkeit sowie die sensibilisierende Wirkung der Thioglykolsäureester.

Trotz einer Vielzahl von Versuchen konnte bisher die sensibilisierende Wirkung von sauren Haarverformungsmitteln nicht entscheidend verringert werden.

Als Alternative wurden daher mildalkalische (pH = 7,1 bis 9) Haarverformungsmittel vorgeschlagen, die als keratinreduzierenden Wirkstoff Cystein oder dessen Salze enthalten

Diese Haarverformungsmittel weisen jedoch ebenfalls eine Reihe von Nachteilen auf. So besitzt Cystein nur eine schwache Verformungswirksamkeit sowie eine geringe Stabilität. Wenn cysteinhaltige Verformungsmittel auf das Haar aufgebracht werden, wird das Cystein durch den Luftsauerstoff schnell zu in Wasser schwer löslichem Cystin oxidiert, welches sich in Form eines störenden schwer entfernbaren weißen Belages auf dem Haar ablagert (sogenannter "Weißeffekt"

Es wurden bereits eine Vielzahl von Versuchen unternommen um diesen "Weißeffekt" zu vermeiden, wobei in der Regel zur Lösung dieses Problems der Zusatz von Coreduktionsmitteln oder anderer, die Oxidationsanfälligkeit des Cysteins herabsetzender Verbindungen zu Haarverformungsmitteln auf Cysteinbasis empfohlen wurde. Durch diese Maßnahmen konnte das bestehende Problem des "Weißeffektes" jedoch nicht in befriedigendem Maße gelöst werden. Zudem besitzen die bisher vorgeschlagenen Haarverformungsmittel häufig den Nachteil einer unzureichenden Verformungswirkung oder aber eines nicht akzeptierbaren Allergiepotentials.

Der Erfindung liegt daher die Aufgabe zugrunde, ein Mittel zur dauerhaften Haarverformung zur Verfügung zu stellen, das im pH-Bereich von etwa 6,5 bis etwa 9,0 eine schonende und gleichmäßige Umformung ermöglicht, ein möglichst geringes Allergiepotential aufweist und bei dem darüber hinaus keine storenden Ablagerungen oder Beläge auf dem Haar entstenen.

Überraschenderweise wurde nunmehr gefunden, daß mit Mitteln zur dauerhaften Verformung von Haaren auf der Basis von N-Glycyl-L-cystein (I) und/oder L-Cysteinylglycin (II)

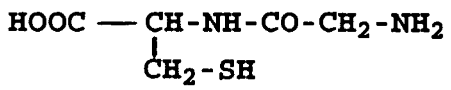 (I)

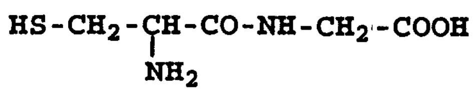 (II)

eine gleichmäßige Umformung sowohl von geschädigtem als auch ungeschädigtem Haar möglich ist, ohne daß hierbei die bei den bisher für die Haarverformung verwendeten haarkeratinreduzierenden Esterverbindungen häufig beobachteten allergischen Hautreaktionen auftreten oder die bei cysteinhaltigen Dauerverformungsmitteln beobachteten störenden Beläge auf dem Haar entstehen.

Gegenstand der vorliegenden Erfindung ist daher ein Mittel zur dauerhaften Verformung von Haaren auf der Basis eines haarkeratinreduzierenden Wirkstoffes, welches als haarkeratinreduzierenden Wirkstoff N-Glycyl-L-cystein und/oder L-Cysteinylglycin enthält.

Zwar ist es möglich N-Glycyl-L-cystein oder L-Cysteinylglycin gemeinsam mit anderen keratinreduzierenden Wirkstoffen - wie zum Beispiel Thioglykolsäure, Thiomilchsäure, 3-Hydroxy-2-mercaptopropionsäure, Cysteamin und Cysteaminderivaten oder Cystein und Cysteinderivaten - zu verwenden, jedoch ist die Verwendung von N-Glycyl-L-cystein und/oder L-Cysteinylglycin als alleinigem keratinreduzierenden Wirkstoff (das heißt ohne Zusatz anderer keratinreduzierender Wirkstoffe) besonders bevorzugt

Das N-Glycyl-L-cystein und/oder L-Cysteinylglycin wird in dem gebrauchsfertigen erfindungsgemäßen Mittel zur dauerhaften Haarverformung in einer Menge von 8 bis 30 Gewichtsprozent, vorzugsweise in einer Menge von 10 bis 25 Gewichtsprozent, eingesetzt.

Das gebrauchsfertige erfindungsgemaße Verformungsmittel besitzt einen pH-Wert vor 6,5 bis 9,0, vorzugsweise von 6,8 bis 8,5

Das Verformungsmittel kann sowohl in Form einer wäßrigen Lösung oder einer Emulsion als auch in verdickter Form auf wäßriger Basis, insbesondere als Gel, Creme oder Paste vorliegen

Selbstverständlich kann das Verformungsmittel alle für derartige Mittel üblichen und bekannten Zusatzstoffe, zum Beispiel Verdickungsmittel wie beispielsweise Kaolin, Bentonit, Fettsäuren, höhere Fettalkohole, Stärke, Polyacrylsäure und deren Derivate, Cellulose-derivate, Alginate, Vaseline oder Paraffinöl; Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quaternäre Ammoniumsalze, Alkylbetaine, oxethylierte Alkylpehnole, Fettsäurealkanolamide oder oxethylierte Fettsäureester; ferner Trübungsmittel, wie zum Beispiel Polyethylenglykolester; oder Alkohole, wie beispielsweise Ethanol, Propanol, Isopropanol oder Glycerin; Lösungsvermittler; Stabilisatoren; Puffersubstanzen; Parfümöle, Farbstoffe sowie haarkonditionierende und haarpflegende Bestandteile, wie zum Beispiel kationische Polymere, Lanolinderivate, Cholesterin, Pantothensäure oder Betain, enthalten. Die erwähnten Bestandteile werden in den für solche Zwecke üblichen Mengen verwendet, zum Beispiel die Netzmittel und Emulgatoren in Konzentrationen von 0,2 bis 30 Gewichtsprozent, wahrend die Verdickungsmittel in einer Menge von 0,5 bis 20 Gewichtsprozent in diesem Mittel enthalten sein können.

Weiterhin können diesem Mittel zur Wirkungssteigerung sogenannte Quell- und Penetrationsstoffe, wie zum Beispiel Dipropylenglykolmonomethylether, 2-Pyrrolidon oder Imidazolidin-2-on, in einer Menge von 2 bis 30 Gewichtsprozent sowie zur Vermeidung einer Überkrausung der Haare Dithioverbindungen, beispielsweise Dithiodiglykolsäure, Dithiodimilchsäure oder deren Salze, zugesetzt werden.

Durch Variation des pH-Wertes dann ein Mittel zur Verfügung gestellt werden, das universell für jede Haarstruktur, gegebenenfalls unter zusätzlicher Wärmeeinwirkung, geeignet ist. Das Mittel bewirkt eine elastische, dauerhafte, gleichmäßige Umformung vom Haaransatz bis zur Haarspitze.

Die dauerhafte Verformung der Haare wird in der üblichen Weise durchgeführt, indem man das Haar bevor und/oder nachdem man es in die gewünschte Form bringt, mit einem Verformungsmittel behandelt, mit Wasser spült, sodann oxidativ nachbehandelt, mit Wasser spült, gegebenenfalls zur Wasserwelle legt und sodann trocknet.

Hierbei kann das Haar zunächst mit einem Shampoo gewaschen und danach mit Wasser gespült werden. Anschließend wird das handtuchtrockene Haar in einzelne Strähnen aufgeteilt und auf Wickler mit einem Durchmesser von 5 bis 30 Millimetern, bevorzugt 5 bis 15 Millimeter, gewickelt. Sodann wird das Haar mit einer für die Haarverformung ausreichenden Menge, vorzugsweise 60 bis 120 Gramm, des beschriebenen erfindungsgemäßen Verformungsmittels behandelt.

Nach einer für die dauerhafte Verformung des Haares ausreichenden Einwirkungszeit, welche je nach Haarbeschaffenheit, dem pH-Wert und der Verformungswirksamkeit des Verformungsmittels sowie in Anhängigkeit von der Anwendungstemperatur 5 bis 30 Minuten (10 bis 30 Minuten ohne Wärmeeinwirkung; 5 bis 20 Minuten mit Wärmeeinwirkung) beträgt, wird das Haar mit Wasser gespült und dann oxidativ nachbehandelt ("fixiert"). Das Nachbehandlungsmittel wird, je nach Haarfülle, vorzugsweise in einer Menge von 80 bis 100 Gramm verwendet.

Für die oxidative Nachbehandlung kann jedes beliebige, bisher für eine derartige Behandlung verwendete, Nachbehandlungsmittel verwendet werden. Beispiele für in solchen Nachbehandlungsmitteln verwendbare Oxidationsmittel sind Kalium- und Natriumbromat, Natriumperborat, Harnstoffperoxid und Hydrogenperoxid. Die Konzentration des Oxidationsmittls ist in Abhängigkeit von der Anwendungszeit (in der Regel 5 bis 15 Minuten) und der Anwendungstemperatur unterschiedlich. Normalerweise liegt das Oxidationsmittel in dem gebrauchsfertigen wäßrigen Nachbehandlungsmittel in einer Konzentration von 0,5 bis 10 Gewichtsprozent vor. Das Mittel für die oxidative Nachbehandlung kann selbstverständlich weitere Stoffe, wie zum Beispiel Netzmittel, Pflegestoffe wie kationaktive Polymere, schwache Säuren, Puffersubstanzen oder Peroxidstabilisatoren, enthalten und in Form einer wäßrigen Lösung, einer Emulsion sowie in verdickter Form auf wäßriger Basis, insbesondere als Creme, Gel oder Paste, vorliegen.

Anschließend werden die Wickler entfernt. Falls erforderlich, kann das abgewickelte Haar nun nochmals oxidativ nachbehandelt werden. Sodann wird das Haar mit Wasser gespült, gegebenenfalls zur Wasserwelle gelegt und schließlich getrocknet.

<u>Beispiele für Haarverformungsmittel</u>

<u>Beispiel 1:</u> Dauerverformungsmittel für gefärbtes Haar

| | |
|---|---|
| 16,0 g | N-Glycyl-L-cystein |
| 3,0 g | Copolymer aus Vinylpyrrolidon und Dimethylaminoethylmethacrylat, mit Diethylsulfat quaternisiert (20-%ige wäßrige Lösung; Handelsname GAFQUAT® 755 der GAF Corp., New York/USA) |
| 2,6 g | Ammoniumhydrogencarbonat |
| 1,5 g | Diethylenglykolmonoethylether |
| 1,5 g | 1,3-Butandiol |
| 1,0 g | 1,2-Propandiol |

| | |
|---|---|
| 1,0 g | Cocobetain |
| 1,0 g | Laurylalkohol, oxethyliert mit 4 Mol Ethylenoxid |
| 0,8 g | Ammoniak (25-%ige wäßrige Lösung) |
| 0,3 g | Vinylpyrrolidon/Styrol-Mischpolymerisat (Handelsname ANTARA® der GAF Corp., New York/USA) |
| 0,3 g | Parfümöl |
| 71,0 g | Wasser |
| 100,0 g | |

Der pH-Wert dieses Dauerverformungsmittels beträgt 8,0.

Durch mehrfache Haarfärbebehandlungen vorgeschädigtes Haar wird gewaschen, mit einem Handtuch frottiert und sodann auf Wickler mit einem Durchmesser von 8 Millimetern gewickelt. Anschließend wird das vorstehend beschriebene Haarverformungsmittel gleichmäßig auf dem gewickelten Haar verteilt. Nach einer Einwirkungszeit von 15 Minuten bei Raumtemperatur wird das gewickelte Haar mit Wasser gespült und mit einem Handtuch leicht angetrocknet. Anschließend wird das gewickelte Haar mit 80 Gramm einer 3-%igen wäßrigen Wasserstoffperoxidlösung oxidativ nachbehandelt.

Nach einer Einwirkungszeit von 5 Minuten werden die Wickler entfernt. Nach weiteren 5 Minuten werden die Haare mit Wasser gespült, mit einem Handtuch frottiert, zur Frisur gelegt und getrocknet.

Als Ergebnis wird eine gleichmäßige, elastische und dauerhafte Verformung der Haare erhalten, wobei keinerlei weiße Beläge auf dem Haar verbleiben.

<u>Beispiel 2:</u> Dauerverformungsmittel für normales Haar

| | |
|---|---|
| 21,4 g | N-Glycyl-L-cystein |
| 5,0 g | Ammoniumhydrogencarbonat |
| 3,0 g | Harnstoff |
| 3,0 g | 1,2-Propylenglykol |
| 1,2 g | Ammoniak (25-%ige wäßrige Lösung) |
| 1,0 g | Glycerin-Polyethylenglykolrizinoleat (Handelsname CREMOPHOR® EL der Firma BASF, Ludwigshafen/BRD) |
| 0,6 g | Parfümöl |
| 0,5 g | Poly(diallyldimethylammoniumchlorid) |
| 0,2 g | Vinylpyrrolidon/Styrol-Mischpolymerisat (Handelsname ANTARA® 430 der GAF Corp., New York/USA). |
| 64,1 g | Wasser |
| 100,0 g | |

Der pH-Wert des Dauerverformungsmittels beträgt 8,5.

Normales, nicht vorgeschädigtes Haar wird gewaschen, mit einem Handtuch frottiert und auf Wickler mit einem Durchmesser von 6 Millimetern gewickelt. Anschließend wird das Haar mit dem vorstehend beschriebenen Haarverformungsmittel gleichmäßig durchfeuchtet. Nach einer Einwirkungszeit von 20 Minuten wird das Haar mit Wasser gründlich gespült und sodann mit 100 Gramm einer 2,5-prozentigen wäßrigen Hydrogenperoxid-Lösung oxidativ nachbehandelt. Nach Entfernung der Wickler werden die Haare erneut mit Wasser gespült, zur Wasserwelle gelegt und anschließend getrocknet.

Das so umgeformte Haar besitzt eine über die gesamte Haarlänge gleichmäßig und lebhafte Krause und weist keinerlei weiße Rückstände auf.

<u>Beispiel 3:</u> Dauerverformungsmittel für hellergefärbtes und blondiertes Haar

| | |
|---|---|
| 12,0 g | L-Cysteinylglycin |
| 2,5 g | Monoethanolamin |
| 2,0 g | Copolymer aus Vinylpyrrolidon und Dimethylaminoethylmethacrylat, mit Diethylsulfat quaternisiert (20-%ige wäßrige Lösung; Handelsname GAFQUAT® 755 der GAF Corp., New York/USA) |
| 2,0 g | Poly(diallyldimethylammoniumchlorid) |
| 0,8 g | Hydriertes Rizinusöl, oxethyliert mit 45 Mol Ethylenoxid (Handelsname CREMOPHOR® RH 410 der Firma BASF, Ludwigshafen/BRD) |
| 0,5 g | Parfümöl |
| 0,3 g | Cocamidopropylbetain |
| 0,3 g | Vinylpyrrolidon/Styrol-Mischpolymerisat (Handelsname ANTARA® 430 der GAF Corp., New York/USA) |
| 79,6 g | Wasser |
| 100,0 g | |

Der pH-Wert dieses Mittels beträgt 8,8.

Das Haar wird in der in Beispiel 1 beschriebenen Weise behandelt, wobei die Einwirkungszeit des Verformungsmittels jedoch nur 12 Minuten beträgt und zur oxidativen Nachbehandlung ein übliches bromathaltiges Fixiermittel verwendet wird.

Das so behandelte Haar verfügt über eine gleichmäßige Krause, deren Locken eine gute Elastizität und Sprungkraft aufweisen. Die bei Cysteindauerwellen auftretende Ablagerung eines weißen Belages wird nicht beobachtet.

Alle in der vorliegenden Anmeldung enthaltenden Prozentangaben stellen, soweit nicht anders angegeben, Gewichtsprozent dar.

**Patentansprüche**

1. Mittel zur dauerhaften Verformung von Haaren auf der Basis eines keratinreduzierenden Wirkstoffes, dadurch gekennzeichnet, daß es als keratinreduzierenden Wirkstoff N-Glycyl-L-cystein und/oder L-Cysteinylglycin enthält.

2. Mittel nach Anspruch 1, dadurch gekennzeichnet, daß es als alleinigen keratinreduzierenden Wirkstoff N-Glycyl-L-cystein und/oder L-Cysteinylglycin enthält.

3. Mittel nach Anspruch 1 oder 2, dadurch gekennzeichnet, daß das N-Glycyl-L-cystein und/oder L-Cysteinylglycin in dem gebrauchsfertigen Mittel in einer Menge von 8 bis 30 Gewichtsprozent enthalten ist.

4. Mittel nach einem der Ansprüche 1 bis 3, dadurch gekennzeichnet, daß der pH-Wert des gebrauchsfertigen Mittels 6,5 bis 9,0 beträgt.

5. Verfahren zur dauerhaften Verformung von Haaren, bei dem man das Haar bevor und/oder nachdem man es in die gewünschte Form bringt mit einem Verformungsmittel behandelt, mit Wasser spült, sodann oxidativ nachbehandelt, mit Wasser spült, gegebenenfalls zur Wasserwelle legt und sodann trocknet, dadurch gekennzeichnet, daß man als Verformungsmittel ein Mittel nach einem der Ansprüche 1 bis 4 verwendet.

6. Verfahren nach Anspruch 5, dadurch gekennzeichnet, daß man das Verformungsmittel 5 bis 30 Minuten lang einwirken läßt.

7. Verfahren nach Anspruch 5 oder 6, dadurch gekennzeichnet, daß man das Verformungsmittel in einer Menge von 60 bis 120 Gramm anwendet.

## Claims

1. Agent for the permanent shaping of hair based on a keratin-reducing active substance, characterized in that it contains N-glycyl-L-cysteine and/or L-cysteinylglycine as the keratin-reducing active substance.

2. Agent according to Claim 1, characterised in that it contains N-glycyl-L-cysteine and/or L-cysteinylglycine as the sole keratin-reducing active substance.

3. Agent according to Claim 1 or 2, characterized in that the N-glycyl-L-cysteine and/or L-cysteinylglycine is present in the ready-for-use agent in an amount of from 8 to 30% by weight.

4. Agent according to any one of Claims 1 to 3, characterized in that the pH value of the ready-for-use agent is from 6.5 to 9.0.

5. Process for the permanent shaping of hair, in which the hair, before and/or after it has been brought into the desired shape, is treated with a shaping agent, rinsed with water, subsequently treated oxidatively, rinsed with water, optionally arranged in a water wave and then dried, characterized in that an agent according to any one of Claims 1 to 4 is used as the shaping agent.

6. Process according to Claim 5, characterized in that the shaping agent is allowed to take effect for from 5 to 30 minutes.

7. Process according to Claim 5 or 6, characterized in that the shaping agent is used in an amount of from 60 to 120 g.

## Revendications

1. Agent de déformation permanente des cheveux, à base d'un principe actif réduisant la kératine, caractérisé en ce qu'il contient comme principe actif sur la kératine de la N-glycyl-L-cystéine et/ou de la L-cystéinylglycine.

2. Agent selon la revendication 1, caractérisé en ce qu'il contient comme princpe actif seul réduisant la kératine de la N-glycyl-L-cystéine et/ou de la L-cystéinylglycine.

3. Agent sel on la revendication 1 ou 2, caractérisé en ce que la N-glycyl-L-cystéine et/ou de la L-cystéinylglycine est contenue dans le produit dans le produit prêt à l'utilisation en une quantité comprise dans la plage allant de 3 à 30% en poids.

4. Agent selon l'une des revendications 1 à 3, caractérisé en ce que la valeur du pH du produit prêt à l'utilisation est compris dans la plage allant de 6,5 à 9.

5. Procédé de déformation permanente des cheveux, pour lequel avant et/ou après que l'on mette les cheveux à la forme souhaitée on traite avec un agent de déformation, on rince à l'eau, on effectue ensuite un post-traitement oxydatif, on rince à l'eau, le cas échéant on passe à grandes eaux, et l'on sèche, caractérisé en ce que l'on utilise comme agent de déformation un agent selon l'une des revendications 1 à 4.

6. Procédé selon la revendication 5, caractérisé en ce que l'on fait agir l'agent de déformation pendant une durée de 5 à 30 minutes.

7. Procédé selon la revendication 5 ou 6, caractérisé en ce que l'on utilise l'agent de déformation en une quantité comprise dans la plage allant de 60 à 120 grammes.